# EUROPÄISCHE PATENTSCHRIFT

(11) **EP 2 694 090 B1**
(45) Veröffentlichungstag und Bekanntmachung des Hinweises auf die Patenterteilung: **16.01.2019**
(21) Anmeldenummer: 12720117.6
(22) Anmeldetag: 10.04.2012
(51) Int. Cl.: A61K 38/16, A61P 35/04

(54) **ARZNEIMITTEL ENTHALTEND REKOMBINANTE MISTELLEKTINE ZUR BEHANDLUNG DES MALIGNEN MELANOMS**
MEDICINE CONTAINING RECOMBINANT MISTELLECTINS FOR TREATING MALIGNANT MELANOMA
MÉDICAMENT COMPRENANT DES LECTINES DE GUI RECOMBINANTES POUR LE TRAITEMENT DU MÉLANOME MALIN

(30) Priorität: 06.04.2011 EP 11161400
(43) Veröffentlichungstag der Anmeldung: 12.02.2014
(73) Patentinhaber: Melema Pharma GmbH, 20148 Hamburg (DE)
(72) Erfinder: LENTZEN, Hans, 51503 Rösrath (DE); WITTHOHN, Klaus, 51491 Overath (DE)
(74) Vertreter: Simandi, Claus
(86) Internationale Anmeldenummer: PCT/EP2012/056479
(87) Internationale Veröffentlichungsnummer: WO 2012/136857

(56) Entgegenhaltungen:
- EP-A1- 0 751 221
- WO-A2-03/054544
- DE-A1- 4 221 836
- DE-A1- 19 752 597
- DE-A1- 19 804 210
- A THIES ET AL: "Low-dose mistletoe lectin-I reduces melanoma growth and spread in a scid mouse xenograft model", BRITISH JOURNAL OF CANCER, Bd. 98, Nr. 1, 15. Januar 2008 (2008-01-15), Seiten 106-112, XP055005642, ISSN: 0007-0920, DOI: 10.1038/sj.bjc.6604106
- THIES A ET AL: "Influence of mistletoe lectins and cytokines induced by them on cell proliferation of human melanoma cells in vitro", TOXICOLOGY, LIMERICK, IR, Bd. 207, Nr. 1, 1. Februar 2005 (2005-02-01), Seiten 105-116, XP004675524, ISSN: 0300-483X, DOI: 10.1016/J.TOX.2004.09.009
- MEYER S ET AL: "Use of phytopharmaceutical agents in dermatology; Indications, therapeutic approaches and side effects", DER HAUTARZT ; ZEITSCHRIFT FÜR DERMATOLOGIE, VENEROLOGIE UND VERWANDTE GEBIETE, SPRINGER, BERLIN, DE, Bd. 56, Nr. 5, 1. Mai 2005 (2005-05-01), Seiten 483-502, XP019319539, ISSN: 1432-1173, DOI: 10.1007/S00105-005-0949-Y
- MENGS U ET AL: "Lektinol, a standardized mistletoe preparation with antitumoral activity", MEDICINAL & AROMATIC PLANTS ABSTRACTS, SCIENTIFIC PUBLISHERS, SCIENTIFIC PUBLISHERS, NEW DELHI - INDIA, Bd. 24, Nr. 1, 1. Februar 2002 (2002-02-01), XP018011351, ISSN: 0250-4367

## Beschreibung

Die Erfindung betrifft ein Arzneimittel und / oder pharmazeutische Zusammensetzung zur Behandlung des malignen Melanoms, vor allem des malignen Melanom Stadium IV, und dessen Verwendung, insbesondere dessen Verwendung in ausgewählten Patientenpopulationen.

Das maligne Melanom (auch: schwarzer Hautkrebs) ist ein schnell und frühzeitig metastasierender Tumor der Melanozyten, Melanin-produzierende Zellen, in der basalen Zellschicht der Epidermis. Der Grad der Erkrankung ist abhängig von dem Grad der Metastasierung in die regionalen Lymphknoten und in entfernte Körperregionen. Das maligne Melanom ist besonders aggressiv und bösartig und ist verantwortlich für nahezu 80 % aller Todesfälle der Hauttumore (Parkin DM, Bray F, Ferlay J, Pisani P. 2005. CA Cancer J Clin 55: 74-108). Das maligne Melanom hat unter den Hauttumoren der Männer die größte Steigerungsrate, bei den Frauen hat es die zweitgrößte Steigerungsrate. Weltweit wird von einer Inzidenz von 160.000 neuen Fällen und 41.000 Todesfällen im Jahr ausgegangen (Parkin et al. 2005 (supra)).

Für 2010 wird in den USA mit 68.000 neuen Fällen und mit 8.700 Todesfällen gerechnet (SEER-Statistik, www.seer.cancer.gov), in Europa sind es 62.000 neue Fälle und 16.600 Todesfälle. In Australien und Neuseeland werden 10.000 neue Fälle und 1.300 Todesfälle erwartet (Parkin et al. 2005 (supra)). Für 2010 wird in den 7 bedeutenderen Pharmamärkten der Welt (USA, Japan, Frankreich, Deutschland, Italien, Spanien, Großbritannien) mit 138.000 und im Jahr 2019 mit etwa 227.000 neuen Fällen gerechnet (Globocan 2002 <http://www-dep.iarc.fr/> [Accessed April 7, 2010], World Population Prospects 2008 <http://esa.un.org/unpp/> [Accessed April 7, 2010]).

Wird das maligne Melanom im frühen Stadium diagnostiziert und behandelt, liegt die 5-Jahresüberlebensrate bei 85 %. Nach der Metastasierung (Nah- und Fernmetastasen) des Melanom sinkt die Überlebensrate drastisch. Eine prospektive Analyse von 8 klinischen Prüfungen der Eastern Cooperative Oncology Group (ECOG) mit 1362 Patienten mit metastasiertem malignen Melanom, die mit Kombichemotherapien behandelt wurden, erbrachte ein medianes Überleben von 6,4 Monaten und ein angenommenes 5-Jahresüberleben von 6%. Signifikante Parameter für ein verkürztes Überleben der Patienten mit metastasiertem Melanom sind schlechter Allgemeinzustand, viszerale Metastasen, Anzahl der betroffenen Organe und erhöhtes LDH (Lactatdehydrogenase) (Manola J, Atkins M, Ibrahim J, Kirkwood J. 2000 J Clin Oncol 18: 3782-93, Balch CM, Gershenwald JE, Soong S -J et al. 2009 J Clin Oncol 27(36): 6199 - 6206, Korn E L, Liu P-Y, Lee S J et al. 2008 J Clin Oncol 26(4): 527 - 534).

Das metastasierende maligne Melanom (sogenanntes Stadium IV) ist in der Regel eine unheilbare Erkrankung (Balch et al 2009 (supra)). Die derzeitige Standardtherapie zur Behandlung von Patienten mit metastasiertem Melanom Stadium IV ist Dacarbazine (DTIC) (Garbe C, Hauschild A, Volkenandt M et al. 2005, Deutsche Leitlinien: Malignes Melanom, www.ado-homepage.de). Dacarbazin ist gut verträglich aber es hat nur einen geringen Vorteil für die Patienten hinsichtlich der Responserate und des Überlebens. Die alleinige Anwendung von Dacarbazin erbringt eine Responserate von 5,3 % - 23%, die aber von kurzer Dauer ist (Huncharek M, Caubet J F & McGarry R. 2001 Melanoma Res 11(1): 75 - 81, Serrone L, Zeuli M & Cognetti F 2000 J Exp Clin Res 19(1): 21 - 34). Es gibt keinen zusätzlichen Beleg durch eine klinische Prüfung der Phase III, dass Dacarbazin das Überleben der Patienten verlängert. Das mediane Überleben nach Dacarbazin in Phase III Prüfungen beträgt ca. 7,5 Monate (Chapman P B, Einhorn L H, Meyers M L et al. 1999 J Clin Oncol 17(9): 2745 - 2751, Middleton M R, Grab J J, Aaronson N et al. 2000 J Clin Oncol 18(1): 158 - 166, Atkins M B, Lotze M T, Dutcher J P et al. 1999 J Clin Oncol 17: 2105 - 2116, Falkson C I, Ibrahim J, Kirkwood J M et al. 1998 J Clin Oncol 16: 1743 - 1751, Avril M F, Aamdal S, Grob J J et al. 2004 J Clin Oncol 22: 1118 - 1125, Flaherty L E, Atkins M, Sosman J et al. 2001 J Clin Oncol 19: 3194 - 3202). Auch andere zytotoxische Substanzen wie z.B. Temozolomid mit einer Responserate (ORR) von 13,5 - 21 %, die Substanzen Carboplatin, Cisplatin und Vindesin (ORR 12 - 26 %) und Paclitaxel und Fotemustin (ORR 7,4 - 24,2%) zeigen Aktivität bei Patienten mit metastasiertem Melanom. Die klinische Wirksamkeit dieser Therapien ist vergleichbar jener von Dacarbazin (Chapman et al. 1999 (supra), Middleton et al. 2000 (supra), Atkins et al. 1999 (supra)). So wird mit Treosulfan in einer 2.-Linien-Therapie nach Dacarbazin ein medianes Überleben von 6,5 Monaten und eine 1-Jahresüberlebensrate von 33,9 % gefunden, verbunden mit 15 - 18 % schwerwiegenden hämatologischen Nebenwirkungen (Neuber K, Reinhold U, Deutschmann A et al 2003 Melanoma Res 13: 81 - 85).

Viele dieser Substanzen werden in Kombinationstherapien angewendet (Polychemotherapie) mit dem Ziel, die Responseraten zu erhöhen und das Überleben der Patienten zu verlängern. Mit den Polychemotherapien konnte zwar die Responserate erhöht werden, jedoch hatte die Therapie keinen Einfluss auf die Überlebensrate (OS) im Vergleich zur alleinigen Anwendung von Dacarbazin (Agarwala S S, Glaspy J, O'Day S J et al. 2002 J Clin Oncol 20: 125 - 133, Eton O, Legha S S, Bedikian A Y et al. 2002 J Clin Oncol 20: 2045 - 2052, Falkson et al 1998 (supra), Avril et al. 2004 (supra)). Zwei Beispiele der Polychemotherapien sind das BHD-Regime (ORR: 12.7 % - 30.4 %) und das DVP-Regime (ORR: 31.4 % - 45 %).

Zusätzlich zur Chemotherapie wird seit einigen Jahren bei Patienten mit metastasiertem malignen Melanom eine Immuntherapie mit dem für diese Therapie zugelassener HochDosis Interleukin-2 (IL-2) angewendet. Berichte mit signifikanten klinischen Effekten sind bekannt, obwohl ausgewählte Patientengruppen betroffen sind (Danson S, Lorigan P, Arance A et al. 2003 J Clin Oncol 21: 2551 - 2557). Die erreichten Tumor-Responseraten (ORR: 16 % - 21.5%), sind jedoch begleitet von extensiven Multiorgantoxizitäten und limitieren somit die Anwendung von IL-2. Ähnlich verhält es sich bei der Anwendung von Hochdosis und mittlerer Dosis von Interferon-alpha (IFN-alpha). Die Behandlung mit GM-CSF schien nur in kleinen Studien und in klinischen Prüfungen der frühen Phase erfolgreich zu sein.

Die Kombination von Chemotherapeutika und Zytokinen (Polychemoimmuntherapie) zeigt partiell höhere Responseraten (ORR) im Vergleich zu den Monotherapien, aber erbringt keine Verbesserung des Überlebens. So zeigte die Kombination von IL-2 und Cisplatin mit 50 % eine hohe Responserate kurzer Dauer, die jedoch von starken Nebenwirkungen (unerwünschten Wirkungen) begleitet waren. Ein Vergleich einer Monotherapie mit Dacarbazin und einer Kombination von Dacarbazin, Cisplatin, IFN- und IL-2 zeigte keinen Unterschied zwischen den beiden Behandlungsarmen (Flaherty et al. 2001 (supra), Danson et al. 2003 (supra), Agarwala et al. 2002 (supra), Eton et al 2002 (supra), Falkson et al 1998 (supra)). Eine Kombination von Chemotherapie (Cisplatin, Vinblastine, Dacarbazin) verbunden mit einer Daueranwendung von Biotherapeutika (Interleukin-2, Interferon alfa-2b und GM-CSF in verschiedenen Regimen) zeigte für Patienten mit metastasiertem malignen Melanom ein medianes Überleben von 14 Monaten. Diese Verlängerung des allgemeinen Überlebens war begleitet von einer großen Anzahl an hämatologischen und nichthämatologischen Nebenwirkungen mit CTC Grad 3 und CTC Grad 4. (O'Day S J, Atkins M B, Boasberg P et al. 2009 J Clin Oncol 27(36): 6207 - 6212).

Betreffend metastasierender Tumore sind die Studienergebnisse von Ipilimumab (BMS, Yervoy®), einem monoklonalen Antikörper, der humanes CTLA-4 erkennt, erfolgreich. Unter Therapie mit Ipilimumab konnte das mediane Überleben der Patienten mit metastasiertem Melanom (Stadium III und Stadium IV) signifikant verlängert werden und zwar auf 10 Monate versus 6,4 Monate in der Kontrollgruppe (Hodi F S, O'Day S J, McDermott D F et al. 2010 N Engl J Med 363 (8): 711 - 723). Dies entspricht einer 1-Jahresüberlebensrate von 45,6 % in der Ipilimumab Gruppe im Vergleich zu 25,3 % in der Kontrollgruppe. Die Nebenwirkungen unter Ipilimumab werden jedoch als sehr schwerwiegend betrachtet. 10 - 15 % der Patienten hatten schwerwiegende immun-bedingte Nebenwirkungen (CTC-Grad 3 und Grad 4) mit Auswirkungen auf der Haut und auf den Darmtrakt (Hodi et al. 2010 (supra)).

Es besteht daher ein großes Bedürfnis Arzneimittel bereitzustellen, die Patientenpopulationen im Stadium III oder IV mit metastasierenden Tumoren besser versorgen oder zumindest die Lebenserwartung signifikant erhöhen, insbesondere beim Versagen einer Standardtherapie.

Mistelextrakte werden seit Jahrhunderten therapeutisch genutzt. Insbesondere in der Krebstherapie sind Mistelpräparate mit unterschiedlichem Erfolg eingesetzt worden (Bocci V 1993 J Biol Regulators and Homeostatic Agents 7(1): 1 - 6; Gabius H-J, Gabius S, Joshi S S et al. 1993 Planta Med 60: 2 - 7; Gabius H-J & Gabius S 1994 PZ 139: 9 - 16; Ganguly C & Das S 1994 Chemotherapy 40: 272 - 278, Hajto T, Hostanska K, Gabius H_J 1989 Cancer Res 49: 4803 - 4808, Hajto T, Hostanska K, Frei K et al. 1990 Cancer Res. 50: 3322 - 3326). Es zeigte sich, dass die therapeutischen Effekte insbesondere durch sogenannte Mistellektine (Viscumine, Viscum album Agglutinine, VAA) vermittelt werden. Den Mistellektinen wird dabei neben einer zytotoxischen Wirkung auch eine unspezifische Immunstimulation zugesprochen, deren positive Effekte zur Therapie von Tumorpatienten genutzt werden. Verschiedene Untersuchungen mit Mistellektinen in vitro (Hajto et al., 1990 (supra); Männel D N, Becker H, Gundt A et al. 1991 Cancer Immunol Immunother 33: 177 - 182; Beuth J, Ko K L, Tunggal L et al. 1993 Drug Res 43: 166 - 169) und in vivo (Hajto T 1986 Oncology 43 suppl 1: 51 - 65; Hajto et al., 1989 (supra), Beuth J, Ko H L, Gabius H-J et al. 1991 In Vivo 5: 29 - 32; Beuth J, Ko H L, Gabius H-J et al. 1992 J Clin Invest 70: 658 - 661), sowie klinische Studien (Beuth et al., 1992 (supra)) zeigten eine erhöhte Freisetzung von inflammmatorischen Zytokinen (TNF-alpha, IL-1, IL-6) und eine Aktivierung von zellulären Komponenten des Immunsystems (TH -Zellen, NK-Zellen).

Durch Analysen des Mistelextraktes konnten bisher drei Mistellektine (ML-I, ML-II, ML-III) mit unterschiedlichen Molekulargewichten und Zuckerbindungsspezifitäten identifiziert werden. Es konnte gezeigt werden, dass der immunstimulierende Effekt des Mistelextrakts auf das ML-I zurückzuführen ist. Das ML-I-Lektin besteht aus jeweils zwei glykosylierten A- und B-Ketten (MLA bzw. MLB). Die A-Kette ist für eine enzymatische Inaktivierung von Ribosomen (Endo Y, Tsurugi K & Franz H 1988 FEBS Lett 231: 378 - 380) verantwortlich ist, während die B-Kette bei der Carbohydratbindung beteiligt ist. Die beiden Ketten sind durch Disulfidbrücken miteinander verknüpft. Die resultierenden Mistellektin-Monomere können sich unter Ausbildung von nicht kovalenten Bindungen zu Dimeren zusammenlagern.

Es ist möglich, das biologisch aktive Mistellektin auch rekombinant herzustellen. EP 0751221 beschreibt die Reindarstellung von Mistellektin-Polypeptiden als strukturell homogene Substanz, wobei ausgehend von den Gensequenzen des Mistellektins rekombinante, hochreine Einzelketten (A-Kette, B-Kette) hergestellt werden, die *in vitro* reassoziiert werden können und so ein rekombinantes Mistellektin-Holoprotein ergeben, das proteinchemisch, enzymatisch und strukturell homogen ist, sogenanntes Aviscuminum. Gemäß EP 0751221 ist das rekombinante Mistellektin-Polypeptid sowohl als Holoprotein, als Teilkette und in Form von Subfragmenten für therapeutische Zwecke geeignet und erfindungsgemäß umfasst.

Bisher wurden rekombinante Mistellektine insbesondere in der Behandlung von Tumorerkrankungen eingesetzt. Die Verwendung von rekombinanten Mistellektinen zur Behandlung von Hautkrebs, insbesondere eines malignen Melanoms auch in der Form eines metastasierenden Tumors ist jedoch nicht im Stand der Technik beschrieben.

Überraschenderweise konnte nunmehr gezeigt werden, dass unter der Therapie mit rekombinanten Mistellektinen das Überleben von Tumorpatienten mit metastasierendem Melanom (Stadium III und IV) signifikant verlängert werden kann bzw. die 1-JahresÜberlebensrate signifikant steigt.

Die Aufgabe der vorliegenden Erfindung besteht darin, ein Arzneimittel und pharmazeutisches Mittel bereitzustellen mit denen Hautkrebs, am Mensch wirksam behandelt werden kann.

Die Aufgabe wird durch die Bereitstellung eines Arzneimittels sowie einer pharmazeutischen Zusammensetzung gelöst, wobei diese rekombinante Mistellektine enthalten zur Behandlung von Hautkrebs, insbesondere malignes Melanom, wobei die rekombinanten Mistellektine die folgenden Aminosäuresequenzen umfassen:

Heterodimer bestehend aus den Sequenzen SEQ ID No. 1 und SEQ ID No. 4, siehe z.B. EP 0 751 221 (sogenanntes Aviscuminum).

Unter "biologischer Aktivität des rekombinanten Mistellektins" wird hier jede biologische Aktivität aus dem Spektrum der gesamten biologischen Aktivitäten des rekombinanten Mistellektins verstanden. Eine derartige Funktion ist z.B. die pharmakologische Wirkung des rekombinanten Mistellektins.

Untersuchungen von ML-I-Monomeren ergaben 25 verschiedene Isoformen, die auf unterschiedliche Kombinationen verschiedener A- und B-Ketten sowie unterschiedliche Glykosylierungszustände der Ketten zurückzuführen sind.

Bevorzugt ist weiterhin, dass die Verwendung von erfindungsgemäßen rekombinanten Mistellektinen bei solchen Patientenpopulationen zum Tragen kommt, die mittels einer Standardtherapie nicht auf Tumorpräparate ansprechen, bzw. Nicht-Responder oder Therapieversager umfassen.

Daher umfasst die Erfindung solche Patienten oder Patientenpopulationen von Nicht-Respondern und Therapieversagern bei Hautkrebs, besonders bevorzugt im Stadium III und IV, bei denen eine Standardtumortherapie nicht erfolgreich ist.

Daher betrifft die Erfindung eine solche Auswahl von Patienten oder Patientenpopulationen, die nach erster Behandlung von Hautkrebs, mit einem Tumorpräparat, wie oben beispielhaft für das maligne Melanom beschrieben, mit den erfindungsgemäßen rekombinanten Mistellektinen behandelt werden. Daher werden bevorzugt solche Patienten oder Patientenpopulationen mit den erfindungsgemäßen rekombinanten Mistellektinen behandelt, die im fortgeschrittenen oder Endstadium einer Tumorerkrankung stehen, wobei eine Metastasierung (Stadium III und IV), bei einem malignen Melanom, eingetreten ist. Von daher ist beispielsweise zu einem malignem Melanom ebenfalls eine Kombinationstherapie eines Patienten erfindungsgemäß umfasst, wobei zuerst ein erstes Anti-Tumormittel, wie beispielsweise Dacarbazin, Dacarbazin kombiniert mit Interferon-alpha, Dacarbazin kombiniert mit Vindesin, Treosulfan kombiniert mit Gemcitabin, Imatinib verabreicht wird, und anschließend alleine oder in Kombination zusätzlich die erfindungsgemäßen rekombinanten Mistellektinen verabreicht werden.

Besonders bevorzugt ist das erfindungsgemäße Arzneimittel zur Behandlung von malignen Melanomen im Stadium III und IV geeignet, da überraschender Weise eine signifikante Lebensverlängerung eines einzelnen Patienten oder einer entsprechenden Patientenpopulation erreicht werden kann.

Dieses Ergebnis ist völlig unerwartet und diese spezielle Eignung und Vorteil kann von einem Tumorarzneimittel per se nicht erwartet werden.

Von daher betrifft das Arzneimittel ein neues Anti-Tumormittel und zwar zur Behandlung von malignen Melanomen, vorzugsweise im Stadium III und IV.

Im Sinne dieser Erfindung ist ein "malignes Melanom" wie vorweg beschrieben zu verstehen, wobei jedoch die Stadien III und IV solche Formen eines malignen Melanoms beschreiben, die erfindungsgemäß eine Metastasierung des Tumors darlegen (siehe z.B. Beschreibung im Pschyrembel ®, De Gruyter Verlag, Berlin).

Die Erfindung betrifft außerdem ein Arzneimittel für die Behandlung des malignen Melanoms, welches das rekombinante Mistellektin-Polypeptid, gegebenenfalls zusammen mit einem pharmazeutisch verträglichen Träger enthält unter Ausbildung einer pharmazeutischen Zusammensetzung. Beispiele für besonders geeignete pharmakologisch verträgliche Träger sind dem Fachmann auf dem Gebiet der Tumorheilkunde bekannt und umfassen gepufferte Kochsalzlösungen, Wasser, u.a., verschiedene Arten von Detergenzien, sterile Lösungen, etc. Arzneimittel, die solche Träger umfassen, können mittels bekannter konventioneller Methoden formuliert werden. Diese Arzneimittel können einem Individuum in einer geeigneten Dosis verabreicht werden. Die Verabreichung kann lokal, oral oder parenteral erfolgen, z.B. intravenös, intraperitoneal, subcutan, intramuskulär, lokal, intranasal, intrabronchial oder intradermal, oder über einen Katheter an einer Stelle in einer Arterie. Die Art der Dosierung wird vom behandelnden Arzt entsprechend den klinischen Faktoren bestimmt. Es ist dem Fachmann bekannt, dass die Art der Dosierung von verschiedenen Faktoren abhängig ist, wie z.B. der Körpergröße bzw. dem Gewicht, der Körperoberfläche, dem Alter, dem Geschlecht oder der allgemeinen Gesundheit des Patienten, aber auch von dem speziell zu verabreichenden Mittel, der Dauer und Art der Verabreichung, und von anderen Medikamenten, die möglicherweise parallel verabreicht werden.

Die pharmazeutische Zusammensetzung, welche die erfindungsgemäßen rekombinanten Mistellektin-Polypeptide umfasst, kann lokal oder systemisch verabreicht werden.

Die pharmazeutische Zusammensetzung wird erfindungsgemäß bei der Behandlung des malignen Melanoms verwendet.

Als vorteilhaft hat sich eine Dosierung der erfindungsgemäßen Mistellektine für die humane Anwendung von 2 - 10 ng/kg (Körpergewicht) erwiesen. Besonders vorteilhaft ist die Dosierung einem Bereich von 3 - 7 ng/kg. Vorzugsweise beträgt die verabreichte Menge 5 ng/kg Körpergewicht. Die bevorzugte nicht auf das Körpergewicht bezogene humane Dosierung beträgt 350 ng.
Das erfindungsgemäße Arzneimittel wird über eine Zeitdauer von mindestens 8 Wochen mit einer Frequenz von 1 x täglich bis 1 x pro Woche appliziert. Vorzugsweise wird das Arzneimittel 2 bis 3 x pro Woche verabreicht, besonders bevorzugt ist 2 x pro Woche.

Daher betrifft die Erfindung ein Verfahren zur Dosierung der erfindungsgemäßen rekombinanten Mistellektine oder des erfindungsgemäßen Arzneimittels, wobei die Dosierung 2 bis 10 ng/kg (Körpergewicht) beträgt. Insbesondere betrifft die Erfindung ein Verfahren zur Dosierung der erfindungsgemäßen rekombinanten Mistellektine oder des erfindungsgemäßen Arzneimittels, wobei die Dosis 200 - 500 ng, inbesondere 350 ng beträgt und mindestens 1 x pro Woche dem Patienten verabreicht wird. Der Patient ist bevorzugt ein Patient im fortgeschrittenen oder Endstadium einer Tumorerkrankung, wobei eine Metastasierung (Stadium III und IV), insbesondere bei einem malignen Melanom, eingetreten ist.

Nachfolgende Beispiele und Figuren dienen zur Erläuterung der Erfindung, ohne jedoch die Erfindung auf diese Beispiele zu beschränken.

### Beispiele und Figuren:

### Beispiel 1 einer Zusammensetzung des Arzneimittels

Lösung zur Injektion: 1 mL Ampulle mit 0,5 mL / 1,0 mL Injektionslösung

| | | |
|---|---|---|
| Aviscuminum | 200 - 500 ng | |
| Natriummonohydrogenphosphat Dihydrat | 2,8 mg | 5,6 mg |
| Natriumdihydrogenphosphat Dihydrat | 0,078 mg | 0,155 mg |
| Natriumchlorid | 3,3 mg | 6,7 mg |
| Polyoxyethylen sorbitan ester (Polysorbat) | 0,1 mg | 0,1 mg |
| Glutaminsäure | 0,1 mg | 0,1 mg |
| Wasser zur Injektion | ad 0,5 ml | ad 1,0 mL |

### Beispiel 2 einer Zusammensetzung des Arzneimittels

Pulver zur Herstellung einer Lösung zur Injektion, 2R Glasvial mit

| | |
|---|---|
| Aviscuminum | 200 - 500 ng |
| Trehalose | 40,0 mg |
| Natriumchlorid | 1,0 mg |
| Tris(hydroxymethyl)aminomethan (TRIS) | 0,6 mg |
| Polyoxyethylen sorbitan ester (Polysorbat) | 0,1 mg |
| Salzsäure zur Einstellung des pH Werts | |

zur Verabreichung wird das Pulver mit 0,5 mL oder 1,0 mL Wasser zur Injektion gelöst.

Im Rahmen einer klinischen Studie sollte nun geprüft werden, ob rekombinantes Mistellektin (Aviscumin, "rML" gemäß EP 0 751 221) bei Patienten mit metastasierendem malignen Melanom Stadium IV nach Versagen der Standardtherapie das Fortschreiten der Erkrankung aufhalten kann und ob das Überleben der Patienten verlängert werden kann. In die Studie eingeschlossen wurden 31 auswertbare Patienten. Obwohl auch eine Veränderung des progressionsfreien Überlebens eintrat, wurde überraschenderweise das Überleben der Patienten signifikant erhöht. Das mediane Überleben der Patienten betrug 11,0 Monate, die 1-Jahresüberlebensrate betrug 45,0 %. Die Verlängerung des Überlebens war unabhängig von der Anzahl der Vortherapien und unabhängig vom Allgemeinbefinden (ECOG-Status 0 oder 1). Die 1-Jahresüberlebensrate einer vergleichbaren Kontrollgruppe, die entsprechend den Kriterien Geschlecht, Hirnmetastasen vorhanden / nicht vorhanden, Art der Metastasen (viszeral / nicht viszeral) und Allgemeinbefinden (ECOG) nach den Daten von Korn et al. 2008 (supra) kalkuliert werden kann, beträgt 33,1 %. Es traten keine Nebenwirkungen mit einen Schweregrad von >2 entsprechend den CTC-Kriterien unter der Therapie von rML auf. Damit ist die Anwendung von rML sehr gut verträglich.

**Tabelle 1: Demographische Daten (Fachbegriffe in Englisch)**

| Patients, n=31 | | | |
|---|---|---|---|
| Sex | n (%) | Male | 16 (51.6) |
| | | female | 15 (48.4) |
| ECOG | n (%) | 0 | 17 (54.8) |
| | | 1 | 14 (45.2) |
| Age | Mean | | 65.32 |
| (yrs) | SD | | 13.53 |
| | Median | | 67.00 |
| Range | | | 20 - 86 |
| Weight (kg) | Mean | | 76.53 |
| | SD | | 12.42 |
| | Median | | 77.50 |
| Type of melanoma | n (%) | cutaneous | 26 (83.9) |
| | | mucosal | 3 (9.7) |
| | | occult | 1 (3.2) |
| | | other | 1 (3.2) |
| No. of metastatic sites | n (%) | 1 | 13 (41.9) |
| | | 2 | 13 (41.9) |
| | | 3 | 4 (12.9) |
| | | 4 | 1 (3.2) |
| LDH (U/L) at BL | Mean | | 262.71 |
| | SD | | 89.17 |
| | Median | | 245.00 |
| LDH elevation | n (%) | yes | 17 (54.8) |
| | | no | 14 (45.2) |

| | | | |
|---|---|---|---|
| ECOG = Eastern Cooperative Oncology Group, LDH = Lactatdehydrogenase | | | |

### Fallbeispiel 1:

Patient, weiblich, Alter: 78 Jahre, malignes Melanom Stadium IV, ECOG: 1,
Metastasen in Lymphknoten und Lunge,
2 Vorbehandlungen mit Dacarbazin,
15 Zyklen (420 Tage) Therapie mit Aviscuminum (rML) 350 ng, 2 x pro Woche, Stabilisierung der Erkrankung (kein Tumorwachstum) über einen Zeitraum von 433 Tagen, Überleben: 453 Tage

### Fallbeispiel 2:

Patient, männlich, Alter: 79 Jahre, malignes Melanom Stadium IV, ECOG: 0,
multiple Metastasen in Leber und Lunge,
5 Vorbehandlungen mit Dacarbazin, Dacarbazin kombiniert mit Interferon-alpha, Dacarbazin kombiniert mit Vindesin, Treosulfan kombiniert mit Gemcitabin, Imatinib 4 Zyklen (112 Tage) Therapie mit Aviscuminum (rML) 350 ng, 2 x pro Woche,
Stabilisierung der Erkrankung (kein Tumorwachstum) über einen Zeitraum von 116 Tagen, Überleben: 435 Tage

### Beschreibung der Figuren:

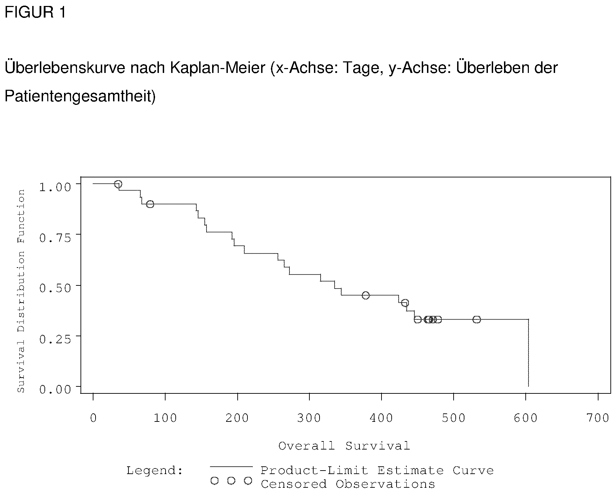

Figur 1 beschreibt die zu den Studiendaten ausgewertete Überlebenskurve nach der Methode von Kaplan-Meier.

## Patentansprüche

1. Arzneimittel enthaltend rekombinantes Mistellektin zur Verwendung in der Behandlung von Hautkrebs am Menschen, wobei das rekombinante Mistellektin aus den Aminosäuresequenzen SEQ ID No. 1 und SEQ ID No. 4 besteht.

2. Arzneimittel zur Verwendung nach Anspruch 1, wobei der Hautkrebs ein malignes Melanom ist.

3. Arzneimittel zur Verwendung nach einem der Ansprüche 1 oder 2, wobei das Arzneimittel ausgewählt ist für Nicht-Responder und Therapieversager einer Standardtumortherapie.

4. Arzneimittel zur Verwendung nach einem der Ansprüche 1 bis 3, wobei das Arzneimittel ausgewählt ist für die Behandlung der Stadien III und IV eines metastasierenden Tumors oder Hautkrebs, insbesondere eines malignen Melanom.

5. Arzneimittel zur Verwendung nach einem der Ansprüche 1 bis 4, wobei das Arzneimittel nach einer ersten Tumorstandardtherapie verwendet wird.

6. Arzneimittel zur Verwendung nach einem der Ansprüche 1 bis 5, gegebenenfalls zusammen mit einem pharmazeutisch verträglichen Träger.

7. Arzneimittel zur Verwendung nach einem der Ansprüche 1 bis 6, wobei das Arzneimittel in einer Dosierung in einem Bereich von 3 - 7 ng rekombinantem Mistellektin pro kg Körpergewicht eingesetzt wird.

8. Arzneimittel zur Verwendung nach Anspruch 7, wobei das Arzneimittel in einer Dosierung von 5 ng rekombinantem Mistellektin pro kg Körpergewicht eingesetzt wird.

9. Arzneimittel zur Verwendung nach einem der Ansprüche 1 bis 6, wobei unabhängig vom Körpergewicht die Dosis an rekombinantem Mistellektin 200 - 500 ng beträgt.

10. Arzneimittel zur Verwendung nach Anspruch 10, wobei unabhängig vom Körpergewicht die Dosis an rekombinantem Mistellektin 350 ng beträgt.

11. Arzneimittel zur Verwendung nach einem der vorgehenden Ansprüche, wobei das Arzneimittel mindestens einmal pro Woche, vorzugsweise 2 oder 3 mal pro Woche verabreicht wird.

## Claims

1. A medicine containing recombinant mistletoe lectin for use in treating skin cancer in humans, wherein the recombinant mistletoe lectin consists of the amino acid sequences of SEQ ID No. 1 and SEQ ID No. 4.

2. The medicine for use according to claim 1, wherein the skin cancer is a malignant melanoma.

3. The medicine for use according to either one of claims 1 or 2, wherein the medicine is selected for non-responders and therapeutic failures of a standard tumour therapy.

4. The medicine for use according to any one of claims 1 to 3, wherein the medicine is selected for the treatment of stages III and IV of a metastatic tumour or skin cancer, in particular of a malignant melanoma.

5. The medicine for use according to any one of claims 1 to 4, wherein the medicine is used after a first standard treatment for tumours.

6. The medicine for use according to any one of claims 1 to 5, possibly together with a pharmaceutically acceptable carrier.

7. The medicine for use according to any one of claims 1 to 6, wherein the medicine is used in a dosage in a range of 3-7 ng recombinant mistletoe lectin per kg body weight.

8. The medicine for use according to claim 7, wherein the medicine is used in a dosage of 5 ng recombinant mistletoe lectin per kg body weight.

9. The medicine for use according to any one of claims 1 to 6, wherein the dosage of recombinant mistletoe lectin is 200-500 ng, independently of body weight.

10. The medicine for use according to claim 10, wherein the dosage of recombinant mistletoe lectin is 350 ng, independently of body weight.

11. The medicine for use according to any one of the preceding claims, wherein the medicine is administered at least once a week, preferably two or three times per week.

## Revendications

1. Médicament contenant de la lectine de gui recombinante pour l'utilisation dans le traitement du cancer de la peau chez l'humain, la lectine de gui recombinante étant constituée des séquences d'acides aminés SEQ ID N° 1 et SEQ ID N° 4.

2. Médicament pour l'utilisation selon la revendication 1, dans lequel le cancer de la peau est un mélanome malin.

3. Médicament pour l'utilisation selon l'une des revendications 1 ou 2, où le médicament est choisi pour des personnes non répondantes et des personnes en situation d'échec de la thérapie d'une thérapie tumorale usuelle.

4. Médicament pour l'utilisation selon l'une des revendications 1 à 3, où le médicament est choisi pour le traitement des stades III et IV d'une tumeur se métastasant ou du cancer de la peau, en particulier un mélanome malin.

5. Médicament pour l'utilisation selon l'une des revendications 1 à 4, où le médicament est employé après une première thérapie tumorale usuelle.

6. Médicament pour l'utilisation selon l'une des revendications 1 à 5, éventuellement accompagné d'un support pharmaceutiquement compatible.

7. Médicament pour l'utilisation selon l'une des revendications 1 à 6, où le médicament est mis en oeuvre dans un dosage dans une plage de 3 à 7 ng de lectine de gui recombinante par kg de poids corporel.

8. Médicament pour l'utilisation selon la revendication 7, où le médicament est mis en oeuvre dans un dosage de 5 ng de lectine de gui recombinante par kg de poids corporel.

9. Médicament pour l'utilisation selon l'une des revendications 1 à 6, dans lequel la dose en lectine de gui recombinante est de 200 à 500 ng indépendamment du poids corporel.

10. Médicament pour l'utilisation selon la revendication 10, dans lequel la dose en lectine de gui recombinante est de 350 ng indépendamment du poids corporel.

11. Médicament pour l'utilisation selon l'une des revendications précédentes, où le médicament est administré au moins une fois par semaine, de préférence 2 ou 3 fois par semaine.


### SEQUENCE LISTING

<110> Cytavis Biopharma GmbH
<120> Arzneimittel enthaltend rekombinante Mistellektine zur Behandlung des
   malignen Melanoms
   <130> CYT22
<160> 12
<170> PatentIn version 3.3
<210> 1
   <211> 253
   <212> PRT
   <213> Artificial
<220>
   <223> EP0751221 recombinant Protein
<220>
   <221> misc_feature
   <222> (1)..(1)
   <223> xaa can be Met or can be deleted
<220>
   <221> misc_feature
   <222> (5)..(5)
   <223> xaa can be Ile or Leu
<220>
   <221> misc_feature
   <222> (16)..(16)
   <223> xaa can be Glu or Asp
<400> 1
   Xaa Tyr Glu Arg Xaa Arg Leu Arg Val Thr His Gln Thr Thr Gly Xaa
   1 5 10 15
   Glu Tyr Phe Arg Phe Ile Thr Leu Leu Arg Asp Tyr Val Ser Ser Gly
   20 25 30
   Ser Phe Ser Asn Glu Ile Pro Leu Leu Arg Gln Ser Thr Ile Pro Val
   35 40 45
   Ser Asp Ala Gln Arg Phe Val Leu Val Glu Leu Thr Asn Gln Gly Gly
   50 55 60
   Asp Ser Ile Thr Ala Ala Ile Asp Val Thr Asn Leu Tyr Val Val Ala
   65 70 75 80
   Tyr Gln Ala Gly Asp Gln Ser Tyr Phe Leu Arg Asp Ala Pro Arg Gly
   85 90 95
   Ala Glu Thr His Leu Phe Thr Gly Thr Thr Arg Ser Ser Leu Pro Phe
   100 105 110
   Asn Gly Ser Tyr Pro Asp Leu Glu Arg Tyr Ala Gly His Arg Asp Gln
   115 120 125
   Ile Pro Leu Gly Ile Asp Gln Leu Ile Gln Ser Val Thr Ala Leu Arg
   130 135 140
   Phe Pro Gly Gly Ser Thr Arg Thr Gln Ala Arg Ser Ile Leu Ile Leu
   145 150 155 160
   Ile Gln Met Ile Ser Glu Ala Ala Arg Phe Asn Pro Ile Leu Trp Arg
   165 170 175
   Ala Arg Gln Tyr Ile Asn Ser Gly Ala Ser Phe Leu Pro Asp Val Tyr
   180 185 190
   Met Leu Glu Leu Glu Thr Ser Trp Gly Gln Gln Ser Thr Gln Val Gln
   195 200 205
   His Ser Thr Asp Gly Val Phe Asn Asn Pro Ile Arg Leu Ala Ile Pro
   210 215 220
   Pro Gly Asn Phe Val Thr Leu Thr Asn Val Arg Asp Val Ile Ala Ser
   225 230 235 240
   Leu Ala Ile Met Leu Phe Val Cys Gly Glu Arg Pro Ser
   245 250
<210> 2
   <211> 256
   <212> PRT
   <213> Artificial
<220>
   <223> EP1051495 recombinant Protein
<220>
   <221> misc_feature
   <222> (1)..(1)
   <223> xaa can be Met or can be deleted
<220>
   <221> misc_feature
   <222> (16)..(16)
   <223> xaa can be Asp or Glu
<220>
   <221> misc_feature
   <222> (64)..(64)
   <223> xaa can be Gly or Gln
<220>
   <221> misc_feature
   <222> (67)..(67)
   <223> Xaa can be Ile or val
<220>
   <221> misc_feature
   <222> (76)..(76)
   <223> xaa can be Leu or Ala
<220>
   <221> misc_feature
   <222> (108)..(108)
   <223> xaa can be Asp-Arg or can be deleted
<220>
   <221> misc_feature
   <222> (114)..(114)
   <223> xaa can be Asn or Thr
<220>
   <221> misc_feature
   <222> (118)..(118)
   <223> xaa can be Pro or Thr
<220>
   <221> misc_feature
   <222> (135)..(135)
   <223> Xaa can be Asp or Glu
<220>
   <221> misc_feature
   <222> (142)..(142)
   <223> xaa can be Ser or Thr
<220>
   <221> misc_feature
   <222> (146)..(146)
   <223> xaa can be Phe or Tyr
<220>
   <221> misc_feature
   <222> (153)..(153)
   <223> xaa can be Ala or Thr
<220>
   <221> misc_feature
   <222> (178)..(178)
   <223> xaa can be Ala or Tyr
<220>
   <221> misc_feature
   <222> (181)..(181)
   <223> xaa can be Tyr or Asp
<220>
   <221> misc_feature
   <222> (186)..(186)
   <223> xaa can be Ala or Glu
<220>
   <221> misc_feature
   <222> (192)..(192)
   <223> xaa can be val or Met
<220>
   <221> misc_feature
   <222> (220)..(220)
   <223> xaa can be Ile or Phe
<220>
   <221> misc_feature
   <222> (225)..(226)
   <223> xaa can be Pro-Ser or Pro-Thr
<220>
   <221> misc_feature
   <222> (233)..(233)
   <223> xaa can be Thr or Ser
<220>
   <221> misc_feature
   <222> (237)..(237)
   <223> xaa can be Asp or Ser
<220>
   <221> misc_feature
   <222> (255)..(256)
   <223> xaa can be Ser-Ser or can be deleted
<400> 2
   Xaa Tyr Glu Arg Leu Arg Leu Arg Val Thr His Gln Thr Thr Gly Xaa
   1               5                   10                  15
   Glu Tyr Phe Arg Phe Ile Thr Leu Leu Arg Asp Tyr Val Ser Ser Gly
               20                  25                  30
   Ser Phe Ser Asn Glu Ile Pro Leu Leu Arg Gln Ser Thr Ile Pro Val
           35                  40                  45
   Ser Asp Ala Gln Arg Phe Val Leu Val Glu Leu Thr Asn Gln Gly Xaa
       50                  55                  60
   Asp Ser Xaa Thr Ala Ala Ile Asp Val Thr Asn Xaa Tyr Val Val Ala
   65                  70                  75                  80
   Tyr Gln Ala Gly Asp Gln Ser Tyr Phe Leu Arg Asp Ala Pro Arg Gly
                   85                  90                  95
   Ala Glu Thr His Leu Phe Thr Gly Thr Thr Arg Xaa Ser Ser Leu Pro
               100                 105                 110
   Phe Xaa Gly Ser Tyr Xaa Asp Leu Glu Arg Tyr Ala Gly His Arg Asp
           115                 120                 125
   Gln Ile Pro Leu Gly Ile Xaa Gln Leu Ile Gln Ser Val Xaa Ala Leu
       130                 135                 140
   Arg Xaa Pro Gly Gly Ser Thr Arg Xaa Gln Ala Arg Ser Ile Leu Ile
   145                 150                 155                 160
   Leu Ile Gln Met Ile Ser Glu Ala Ala Arg Phe Asn Pro Ile Leu Trp
                   165                 170                 175
   Arg Xaa Arg Gln Xaa Ile Asn Ser Gly Xaa Ser Phe Leu Pro Asp Xaa
               180                 185                 190
   Tyr Met Leu Glu Leu Glu Thr Ser Trp Gly Gln Gln Ser Thr Gln Val
           195                 200                 205
   Gln His Ser Thr Asp Gly Val Phe Asn Asn Pro Xaa Arg Leu Ala Ile
       210                 215                 220
   Xaa Xaa Gly Asn Phe Val Thr Leu Xaa Asn Val Arg Xaa Val Ile Ala
   225                 230                 235                 240
   Ser Leu Ala Ile Met Leu Phe Val Cys Gly Glu Arg Pro Ser Xaa Xaa
                   245                 250                 255
<210> 3
   <211> 257
   <212> PRT
   <213> Artificial
<220>
   <223> EP1051495 recombinant Protein
<220>
   <221> misc_feature
   <222> (1)..(1)
   <223> xaa can be Met or can be deleted
<400> 3
   Xaa Tyr Glu Arg Leu Arg Leu Arg Val Thr His Gln Thr Thr Gly Asp
   1               5                   10                  15
   Glu Tyr Phe Arg Phe Ile Thr Leu Leu Arg Asp Tyr Val Ser Ser Gly
               20                  25                  30
   Ser Phe Ser Asn Glu Ile Pro Leu Leu Arg Gln Ser Thr Ile Pro Val
           35                  40                  45
   Ser Asp Ala Gln Arg Phe Val Leu Val Glu Leu Thr Asn Gln Gly Gln
       50                  55                  60
   Asp Ser Ile Thr Ala Ala Ile Asp Val Thr Asn Ala Tyr Val Val Ala
   65                  70                  75                  80
   Tyr Gln Ala Gly Asp Gln Ser Tyr Phe Leu Arg Asp Ala Pro Arg Gly
                   85                  90                  95
   Ala Glu Thr His Leu Phe Thr Gly Thr Thr Arg Asp Arg Ser Ser Leu
               100                 105                 110
   Pro Phe Thr Gly Ser Tyr Thr Asp Leu Glu Arg Tyr Ala Gly His Arg
           115                 120                 125
   Asp Gln Ile Pro Leu Gly Ile Glu Gln Leu Ile Gln Ser Val Ser Ala
       130                 135                 140
   Leu Arg Tyr Pro Gly Gly Ser Thr Arg Ala Gln Ala Arg Ser Ile Leu
   145                 150                 155                 160
   Ile Leu Ile Gln Met Ile Ser Glu Ala Ala Arg Phe Asn Pro Ile Leu
                   165                 170                 175
   Trp Arg Tyr Arg Gln Asp Ile Asn Ser Gly Glu Ser Phe Leu Pro Asp
               180                 185                 190
   Met Tyr Met Leu Glu Leu Glu Thr Ser Trp Gly Gln Gln Ser Thr Gln
   195 200 205
   Val Gln His Ser Thr Asp Gly Val Phe Asn Asn Pro Phe Arg Leu Ala
   210 215 220
   Ile Ser Thr Gly Asn Phe Val Thr Leu Ser Asn Val Arg Ser Val Ile
   225 230 235 240
   Ala Ser Leu Ala Ile Met Leu Phe Val Cys Gly Glu Arg Pro Ser Ser
   245 250 255
   Ser
<210> 4
   <211> 264
   <212> PRT
   <213> Artificial
<220>
   <223> EP0751221 recombinant Protein
<220>
   <221> misc_feature
   <222> (1)..(1)
   <223> xaa can be Met or can be deleted
<400> 4
   Xaa Asp Asp Val Thr Cys Ser Ala Ser Glu Pro Thr Val Arg Ile Val
   1 5 10 15
   Gly Arg Asn Gly Met Cys Val Asp Val Arg Asp Asp Asp Phe Arg Asp
   20 25 30
   Gly Asn Gln Ile Gln Leu Trp Pro Ser Lys Ser Asn Asn Asp Pro Asn
   35 40 45
   Gln Leu Trp Thr Ile Lys Arg Asp Gly Thr Ile Arg Ser Asn Gly Ser
   50 55 60
   Cys Leu Thr Thr Tyr Gly Tyr Thr Ala Gly Val Tyr Val Met Ile Phe
   65 70 75 80
   Asp Cys Asn Thr Ala Val Arg Glu Ala Thr Leu Trp Gln Ile Trp Gly
   85 90 95
   Asn Gly Thr Ile Ile Asn Pro Arg Ser Asn Leu Val Leu Ala Ala Ser
   100 105 110
   Ser Gly Ile Lys Gly Thr Thr Leu Thr Val Gln Thr Leu Asp Tyr Thr
   115 120 125
   Leu Gly Gln Gly Trp Leu Ala Gly Asn Asp Thr Ala Pro Arg Glu Val
       130                 135                 140
   Thr Ile Tyr Gly Phe Arg Asp Leu Cys Met Glu Ser Asn Gly Gly Ser
   145                 150                 155                 160
   Val Trp Val Glu Thr Cys Val Ser Ser Gln Lys Asn Gln Arg Trp Ala
                   165                 170                 175
   Leu Tyr Gly Asp Gly Ser Ile Arg Pro Lys Gln Asn Gln Asp Gln Cys
               180                 185                 190
   Leu Thr Cys Gly Arg Asp Ser Val Ser Thr Val Ile Asn Ile Val Ser
           195                 200                 205
   Cys Ser Ala Gly Ser Ser Gly Gln Arg Trp Val Phe Thr Asn Glu Gly
       210                 215                 220
   Ala Ile Leu Asn Leu Lys Asn Gly Leu Ala Met Asp Val Ala Gln Ala
   225                 230                 235                 240
   Asn Pro Lys Leu Arg Arg Ile Ile Ile Tyr Pro Ala Thr Gly Lys Pro
                   245                 250                 255
   Asn Gln Met Trp Leu Pro Val Pro
               260
<210> 5
   <211> 268
   <212> PRT
   <213> Artificial
<220>
   <223> EP0751221 recombinant Protein
<220>
   <221> misc_feature
   <222> (1)..(1)
   <223> xaa can be Met or can be deleted
<400> 5
   Xaa Asp Asp Val Thr Cys Ser Ala Ser Glu Pro Thr Val Arg Ile Val
   1               5                   10                  15
   Gly Arg Asn Gly Met Cys Val Asp Val Arg Asp Asp Asp Phe Arg Asp
               20                  25                  30
   Gly Asn Gln Ile Gln Leu Trp Pro Ser Lys Ser Asn Asn Asp Pro Asn
           35                  40                  45
   Gln Leu Trp Thr Ile Lys Arg Asp Gly Thr Ile Arg Ser Asn Gly Ser
       50                  55                  60
   Cys Leu Thr Thr Tyr Gly Tyr Thr Ala Gly Val Tyr Val Met Ile Phe
   65 70 75 80
   Asp Cys Asn Thr Ala Val Arg Glu Ala Thr Leu Trp Gln Ile Trp Gly
   85 90 95
   Asn Gly Thr Ile Ile Asn Pro Arg Ser Asn Leu Val Leu Ala Ala Ser
   100 105 110
   Ser Gly Ile Lys Gly Thr Thr Leu Thr Val Gln Thr Leu Asp Tyr Thr
   115 120 125
   Leu Gly Gln Gly Trp Leu Ala Gly Asn Asp Thr Ala Pro Arg Glu Val
   130 135 140
   Thr Ile Tyr Gly Phe Arg Asp Leu Cys Met Glu Ser Asn Gly Gly Ser
   145 150 155 160
   Val Trp Val Glu Thr Cys Val Ser Ser Gln Lys Asn Gln Arg Trp Ala
   165 170 175
   Leu Tyr Gly Asp Gly Ser Ile Arg Pro Lys Gln Asn Gln Asp Gln Cys
   180 185 190
   Leu Thr Cys Gly Arg Asp Ser Val Ser Thr Val Ile Asn Ile Val Ser
   195 200 205
   Cys Ser Ala Gly Ser Ser Gly Gln Arg Trp Val Phe Thr Asn Glu Gly
   210 215 220
   Ala Ile Leu Asn Leu Lys Asn Gly Leu Ala Met Asp Val Ala Gln Ala
   225 230 235 240
   Asn Pro Lys Leu Arg Arg Ile Ile Ile Tyr Pro Ala Thr Gly Lys Pro
   245 250 255
   Asn Gln Met Trp Leu Pro Val Pro Gly Gly Tyr His
   260 265
<210> 6
   <211> 265
   <212> PRT
   <213> Artificial
<220>
   <223> EP1051495 recombinant Protein
<220>
   <221> misc_feature
   <222> (1)..(1)
   <223> xaa can be Met or can be deleted
<220>
   <221> misc_feature
   <222> (19)..(19)
   <223> xaa can be Asn or Ser
<220>
   <221> misc_feature
   <222> (22)..(22)
   <223> xaa can be Cys or Arg
<220>
   <221> misc_feature
   <222> (57)..(57)
   <223> xaa can be Gly or Asn
<220>
   <221> misc_feature
   <222> (96)..(96)
   <223> xaa can be Gly or Asn
<220>
   <221> misc_feature
   <222> (158)..(158)
   <223> xaa can be Gly or Gln
<220>
   <221> misc_feature
   <222> (167)..(167)
   <223> xaa can be Val or Asp
<220>
   <221> misc_feature
   <222> (171)..(171)
   <223> xaa can be Gln or Lys
<220>
   <221> misc_feature
   <222> (174)..(175)
   <223> xaa can be Gly or can be deleted or can be Gly-Arg or Gly-Lys or Arg or
   Lys
<220>
   <221> misc_feature
   <222> (196)..(196)
   <223> xaa can be Cys or val or ser
<220>
   <221> misc_feature
   <222> (212)..(213)
   <223> xaa can be Ala-Ala or Ala-Gly or Gly-Ala or Gly-Gly
<220>
   <221> misc_feature
   <222> (215)..(216)
   <223> xaa can be Ser-Ser or Ser-Gly or Gly-Ser or Gly-Gly
<220>
   <221> misc_feature
   <222> (225)..(225)
   <223> xaa can be Gly or Tyr
<220>
   <221> misc_feature
   <222> (232)..(236)
   <223> xaa232 can be Asn, Ser, Thr or Lys, xaa233 can be Ser or Gly, xaa234 can
   be Leu or Pro, Xaa235 can be Ala or Met, Xaa 236 can be Met or Val
<220>
   <221> misc_feature
   <222> (265)..(265)
   <223> xaa can be Pro or Phe
<400> 6
   Xaa Asp Asp Val Thr Cys Ser Ala Ser Glu Pro Thr Val Arg Ile Val
   1               5                   10                  15
   Gly Arg Xaa Gly Met Xaa Val Asp Val Arg Asp Asp Asp Phe His Asp
               20                  25                  30
   Gly Asn Gln Ile Gln Leu Trp Pro Ser Lys Ser Asn Asn Asp Pro Asn
           35                  40                  45
   Gln Leu Trp Thr Ile Lys Arg Asp Xaa Thr Ile Arg Ser Asn Gly Ser
       50                  55                  60
   Cys Leu Thr Thr Tyr Gly Tyr Thr Ala Gly Val Tyr Val Met Ile Phe
   65                  70                  75                  80
   Asp Cys Asn Thr Ala Val Arg Glu Ala Thr Ile Trp Gln Ile Trp Xaa
                   85                  90                  95
   Asn Gly Thr Ile Ile Asn Pro Arg Ser Asn Leu Val Leu Ala Ala Ser
               100                 105                 110
   Ser Gly Ile Lys Gly Thr Thr Leu Thr Val Gln Thr Leu Asp Tyr Thr
           115                 120                 125
   Leu Gly Gln Gly Trp Leu Ala Gly Asn Asp Thr Ala Pro Arg Glu Val
       130                 135                 140
   Thr Ile Tyr Gly Phe Arg Asp Leu Cys Met Glu Ser Asn Xaa Gly Ser
   145                 150                 155                 160
   Val Trp Val Glu Thr Cys Xaa Ser Ser Gln Xaa Asn Gln Xaa Xaa Trp
                   165                 170                 175
   Ala Leu Tyr Gly Asp Gly Ser Ile Arg Pro Lys Gln Asn Gln Asp Gln
               180                 185                 190
   Cys Leu Thr Xaa Gly Arg Asp Ser Val Ser Thr Val Ile Asn Ile Val
           195                 200                 205
   Ser Cys Ser Xaa Xaa Ser Xaa Xaa Gln Arg Trp Val Phe Thr Asn Glu
       210                 215                 220
   Xaa Ala Ile Leu Asn Leu Lys Xaa Xaa Xaa Xaa Xaa Asp Val Ala Gln
   225                 230                 235                 240
   Ala Asn Pro Lys Leu Arg Arg Ile Ile Ile Tyr Pro Ala Thr Gly Lys
                   245                 250                 255
   Pro Asn Gln Met Trp Leu Pro Val Xaa
               260                 265
<210> 7
   <211> 264
   <212> PRT
   <213> Artificial
<220>
   <223> EP1051495 recombinant Protein
<220>
   <221> misc_feature
   <222> (1)..(1)
   <223> xaa can be Met or can be deleted
<400> 7
   Xaa Asp Asp Val Thr Cys Ser Ala Ser Glu Pro Thr Val Arg Ile Val
   1               5                   10                  15
   Gly Arg Asn Gly Met Cys Val Asp Val Arg Asp Asp Asp Phe His Asp
               20                  25                  30
   Gly Asn Gln Ile Gln Leu Trp Pro Ser Lys Ser Asn Asn Asp Pro Asn
           35                  40                  45
   Gln Leu Trp Thr Ile Lys Arg Asp Gly Thr Ile Arg Ser Asn Gly Ser
       50                  55                  60
   Cys Leu Thr Thr Tyr Gly Tyr Thr Ala Gly Val Tyr Val Met Ile Phe
   65                  70                  75                  80
   Asp Cys Asn Thr Ala Val Arg Glu Ala Thr Ile Trp Gln Ile Trp Gly
                   85                  90                  95
   Asn Gly Thr Ile Ile Asn Pro Arg Ser Asn Leu Val Leu Ala Ala Ser
               100                 105                 110
   Ser Gly Ile Lys Gly Thr Thr Leu Thr Val Gln Thr Leu Asp Tyr Thr
           115                 120                 125
   Leu Gly Gln Gly Trp Leu Ala Gly Asn Asp Thr Ala Pro Arg Glu Val
       130                 135                 140
   Thr Ile Tyr Gly Phe Arg Asp Leu Cys Met Glu Ser Asn Gly Gly Ser
   145                 150                 155                 160
   Val Trp Val Glu Thr Cys Val Ser Ser Gln Gln Asn Gln Arg Trp Ala
                   165                 170                 175
   Leu Tyr Gly Asp Gly Ser Ile Arg Pro Lys Gln Asn Gln Asp Gln Cys
               180                 185                 190
   Leu Thr Cys Gly Arg Asp Ser Val Ser Thr Val Ile Asn Ile Val Ser
   195 200 205
   Cys Ser Ala Gly Ser Ser Gly Gln Arg Trp Val Phe Thr Asn Glu Gly
   210 215 220
   Ala Ile Leu Asn Leu Lys Asn Gly Leu Ala Met Asp Val Ala Gln Ala
   225 230 235 240
   Asn Pro Lys Leu Arg Arg Ile Ile Ile Tyr Pro Ala Thr Gly Lys Pro
   245 250 255
   Asn Gln Met Trp Leu Pro Val Pro
   260
<210> 8
   <211> 265
   <212> PRT
   <213> Artificial
<220>
   <223> EP1051495 recombinant Protein
<220>
   <221> misc_feature
   <222> (1)..(1)
   <223> xaa can be Met or can be deleted
<400> 8
   Xaa Asp Asp Val Thr Cys Ser Ala Ser Glu Pro Thr Val Arg Ile Val
   1 5 10 15
   Gly Arg Asn Gly Met Arg Val Asp Val Arg Asp Asp Asp Phe His Asp
   20 25 30
   Gly Asn Gln Ile Gln Leu Trp Pro Ser Lys Ser Asn Asn Asp Pro Asn
   35 40 45
   Gln Leu Trp Thr Ile Lys Arg Asp Gly Thr Ile Arg Ser Asn Gly Ser
   50 55 60
   Cys Leu Thr Thr Tyr Gly Tyr Thr Ala Gly Val Tyr Val Met Ile Phe
   65 70 75 80
   Asp Cys Asn Thr Ala Val Arg Glu Ala Thr Ile Trp Gln Ile Trp Asp
   85 90 95
   Asn Gly Thr Ile Ile Asn Pro Arg Ser Asn Leu Val Leu Ala Ala Ser
   100 105 110
   Ser Gly Ile Lys Gly Thr Thr Leu Thr Val Gln Thr Leu Asp Tyr Thr
   115 120 125
   Leu Gly Gln Gly Trp Leu Ala Gly Asn Asp Thr Ala Pro Arg Glu Val
   130 135 140
   Thr Ile Tyr Gly Phe Arg Asp Leu Cys Met Glu Ser Asn Gly Gly Ser
   145 150 155 160
   Val Trp Val Glu Thr Cys Asp Ser Ser Gln Lys Asn Gln Gly Lys Trp
   165 170 175
   Ala Leu Tyr Gly Asp Gly Ser Ile Arg Pro Lys Gln Asn Gln Asp Gln
   180 185 190
   Cys Leu Thr Ser Gly Arg Asp Ser Val Ser Thr Val Ile Asn Ile Val
   195 200 205
   Ser Cys Ser Gly Ala Ser Gly Ser Gln Arg Trp Val Phe Thr Asn Glu
   210 215 220
   Gly Ala Ile Leu Asn Leu Lys Asn Gly Leu Ala Met Asp Val Ala Gln
   225 230 235 240
   Ala Asn Pro Lys Leu Arg Arg Ile Ile Ile Tyr Pro Ala Thr Gly Lys
   245 250 255
   Pro Asn Gln Met Trp Leu Pro Val Phe
   260 265
<210> 9
   <211> 265
   <212> PRT
   <213> Artificial
<220>
   <223> EP1051495 recombinant Protein
<220>
   <221> misc_feature
   <222> (1)..(1)
   <223> xaa can be Met or can be deleted
<400> 9
   Xaa Asp Asp Val Thr Cys Ser Ala Ser Glu Pro Thr Val Arg Ile Val
   1 5 10 15
   Gly Arg Ser Gly Met Arg Val Asp Val Arg Asp Asp Asp Phe His Asp
   20 25 30
   Gly Asn Gln Ile Gln Leu Trp Pro Ser Lys Ser Asn Asn Asp Pro Asn
   35 40 45
   Gln Leu Trp Thr Ile Lys Arg Asp Asn Thr Ile Arg Ser Asn Gly Ser
   50 55 60
   Cys Leu Thr Thr Tyr Gly Tyr Thr Ala Gly Val Tyr Val Met Ile Phe
   65 70 75 80
   Asp Cys Asn Thr Ala Val Arg Glu Ala Thr Ile Trp Gln Ile Trp Asp
   85 90 95
   Asn Gly Thr Ile Ile Asn Pro Arg Ser Asn Leu Val Leu Ala Ala Ser
   100 105 110
   Ser Gly Ile Lys Gly Thr Thr Leu Thr Val Gln Thr Leu Asp Tyr Thr
   115 120 125
   Leu Gly Gln Gly Trp Leu Ala Gly Asn Asp Thr Ala Pro Arg Glu Val
   130 135 140
   Thr Ile Tyr Gly Phe Arg Asp Leu Cys Met Glu Ser Asn Gln Gly Ser
   145 150 155 160
   Val Trp Val Glu Thr Cys Asp Ser Ser Gln Lys Asn Gln Gly Lys Trp
   165 170 175
   Ala Leu Tyr Gly Asp Gly Ser Ile Arg Pro Lys Gln Asn Gln Asp Gln
   180 185 190
   Cys Leu Thr Val Gly Arg Asp Ser Val Ser Thr Val Ile Asn Ile Val
   195 200 205
   Ser Cys Ser Gly Ala Ser Gly Ser Gln Arg Trp Val Phe Thr Asn Glu
   210 215 220
   Tyr Ala Ile Leu Asn Leu Lys Ser Gly Leu Ala Met Asp Val Ala Gln
   225 230 235 240
   Ala Asn Pro Lys Leu Arg Arg Ile Ile Ile Tyr Pro Ala Thr Gly Lys
   245 250 255
   Pro Asn Gln Met Trp Leu Pro Val Phe
   260 265
<210> 10
   <211> 265
   <212> PRT
   <213> Artificial
<220>
   <223> EP1051495 recombinant Protein
<220>
   <221> misc_feature
   <222> (1)..(1)
   <223> xaa can be Met or can be deleted
<400> 10
   Xaa Asp Asp Val Thr Cys Ser Ala Ser Glu Pro Thr Val Arg Ile Val
   1               5                   10                  15
   Gly Arg Asn Gly Met Arg Val Asp Val Arg Asp Asp Asp Phe His Asp
               20                  25                  30
   Gly Asn Gln Ile Gln Leu Trp Pro Ser Lys Ser Asn Asn Asp Pro Asn
           35                  40                  45
   Gln Leu Trp Thr Ile Lys Arg Asp Gly Thr Ile Arg Ser Asn Gly Ser
       50                  55                  60
   Cys Leu Thr Thr Tyr Gly Tyr Thr Ala Gly Val Tyr Val Met Ile Phe
   65                  70                  75                  80
   Asp Cys Asn Thr Ala Val Arg Glu Ala Thr Ile Trp Gln Ile Trp Asp
                   85                  90                  95
   Asn Gly Thr Ile Ile Asn Pro Arg Ser Asn Leu Val Leu Ala Ala Ser
               100                 105                 110
   Ser Gly Ile Lys Gly Thr Thr Leu Thr Val Gln Thr Leu Asp Tyr Thr
           115                 120                 125
   Leu Gly Gln Gly Trp Leu Ala Gly Asn Asp Thr Ala Pro Arg Glu Val
       130                 135                 140
   Thr Ile Tyr Gly Phe Arg Asp Leu Cys Met Glu Ser Asn Gly Gly Ser
   145                 150                 155                 160
   Val Trp Val Glu Thr Cys Asp Ser Ser Gln Lys Asn Gln Gly Lys Trp
                   165                 170                 175
   Ala Leu Tyr Gly Asp Gly Ser Ile Arg Pro Lys Gln Asn Gln Asp Gln
               180                 185                 190
   Cys Leu Thr Ser Gly Arg Asp Ser Val Ser Thr Val Ile Asn Ile Val
           195                 200                 205
   Ser Cys Ser Gly Ala Ser Gly Ser Gln Arg Trp Val Phe Thr Asn Glu
       210                 215                 220
   Gly Ala Ile Leu Asn Leu Lys Thr Gly Leu Ala Met Asp Val Ala Gln
   225                 230                 235                 240
   Ala Asn Pro Lys Leu Arg Arg Ile Ile Ile Tyr Pro Ala Thr Gly Lys
                   245                 250                 255
   Pro Asn Gln Met Trp Leu Pro Val Phe
               260                 265
<210> 11
   <211> 265
   <212> PRT
   <213> Artificial
<220>
   <223> EP1051495 recombinant Protein
<220>
   <221> misc_feature
   <222> (1)..(1)
   <223> xaa can be Met or can be deleted
<400> 11
   Xaa Asp Asp Val Thr Cys Ser Ala Ser Glu Pro Thr Val Arg Ile Val
   1               5                   10                  15
   Gly Arg Asn Gly Met Arg Val Asp Val Arg Asp Asp Asp Phe His Asp
               20                  25                  30
   Gly Asn Gln Ile Gln Leu Trp Pro Ser Lys Ser Asn Asn Asp Pro Asn
           35                  40                  45
   Gln Leu Trp Thr Ile Lys Arg Asp Gly Thr Ile Arg Ser Asn Gly Ser
       50                  55                  60
   Cys Leu Thr Thr Tyr Gly Tyr Thr Ala Gly Val Tyr Val Met Ile Phe
   65                  70                  75                  80
   Asp Cys Asn Thr Ala Val Arg Glu Ala Thr Ile Trp Gln Ile Trp Asp
                   85                  90                  95
   Asn Gly Thr Ile Ile Asn Pro Arg Ser Asn Leu Val Leu Ala Ala Ser
               100                 105                 110
   Ser Gly Ile Lys Gly Thr Thr Leu Thr Val Gln Thr Leu Asp Tyr Thr
           115                 120                 125
   Leu Gly Gln Gly Trp Leu Ala Gly Asn Asp Thr Ala Pro Arg Glu Val
       130                 135                 140
   Thr Ile Tyr Gly Phe Arg Asp Leu Cys Met Glu Ser Asn Gly Gly Ser
   145                 150                 155                 160
   Val Trp Val Glu Thr Cys Asp Ser Ser Gln Lys Asn Gln Gly Lys Trp
                   165                 170                 175
   Ala Leu Tyr Gly Asp Gly Ser Ile Arg Pro Lys Gln Asn Gln Asp Gln
               180                 185                 190
   Cys Leu Thr Ser Gly Arg Asp Ser Val Ser Thr Val Ile Asn Ile Val
           195                 200                 205
   Ser Cys Ser Gly Ala Ser Gly Ser Gln Arg Trp Val Phe Thr Asn Glu
       210                 215                 220
   Gly Ala Ile Leu Asn Leu Lys Lys Gly Pro Ala Met Asp Val Ala Gln
   225                 230                 235                 240
   Ala Asn Pro Lys Leu Arg Arg Ile Ile Ile Tyr Pro Ala Thr Gly Lys
                   245                 250                 255
   Pro Asn Gln Met Trp Leu Pro Val Phe
               260                 265
<210> 12
   <211> 265
   <212> PRT
   <213> Artificial
<220>
   <223> EP1051495 recombinant Protein
<220>
   <221> misc_feature
   <222> (1)..(1)
   <223> Xaa can be Met or can be deleted
<400> 12
   Xaa Asp Asp Val Thr Cys Ser Ala Ser Glu Pro Thr Val Arg Ile Val
   1               5                   10                  15
   Gly Arg Asn Gly Met Arg Val Asp Val Arg Asp Asp Asp Phe His Asp
               20                  25                  30
   Gly Asn Gln Ile Gln Leu Trp Pro Ser Lys Ser Asn Asn Asp Pro Asn
           35                  40                  45
   Gln Leu Trp Thr Ile Lys Arg Asp Gly Thr Ile Arg Ser Asn Gly Ser
       50                  55                  60
   Cys Leu Thr Thr Tyr Gly Tyr Thr Ala Gly Val Tyr Val Met Ile Phe
   65                  70                  75                  80
   Asp Cys Asn Thr Ala Val Arg Glu Ala Thr Ile Trp Gln Ile Trp Asp
                   85                  90                  95
   Asn Gly Thr Ile Ile Asn Pro Arg Ser Asn Leu Val Leu Ala Ala Ser
               100                 105                 110
   Ser Gly Ile Lys Gly Thr Thr Leu Thr Val Gln Thr Leu Asp Tyr Thr
           115                 120                 125
   Leu Gly Gln Gly Trp Leu Ala Gly Asn Asp Thr Ala Pro Arg Glu Val
       130                 135                 140
   Thr Ile Tyr Gly Phe Arg Asp Leu Cys Met Glu Ser Asn Gly Gly Ser
   145                 150                 155                 160
   Val Trp Val Glu Thr Cys Asp Ser Ser Gln Lys Asn Gln Gly Lys Trp
                   165                 170                 175
   Ala Leu Tyr Gly Asp Gly Ser Ile Arg Pro Lys Gln Asn Gln Asp Gln
               180                 185                 190
   Cys Leu Thr Ser Gly Arg Asp Ser Val Ser Thr Val Ile Asn Ile Val
           195                 200                 205
   Ser Cys Ser Gly Ala Ser Gly Ser Gln Arg Trp Val Phe Thr Asn Glu
       210                 215                 220
   Gly Ala Ile Leu Asn Leu Lys Asn Ser Leu Met Val Asp Val Ala Gln
   225                 230                 235                 240
   Ala Asn Pro Lys Leu Arg Arg Ile Ile Ile Tyr Pro Ala Thr Gly Lys
                   245                 250                 255
   Pro Asn Gln Met Trp Leu Pro Val Phe
               260                 265